# EUROPEAN PATENT SPECIFICATION

(11) **EP 0 326 381 B1**
(45) Date of publication and mention of the grant of the patent: **08.06.1994**
(21) Application number: 89300751.8
(22) Date of filing: 26.01.1989
(51) Int. Cl.: A61K 37/43

(54) **Use of 6RF in the diagnosis of M. Alzheimer**
Verwendung von 6RF zur Diagnose von M. Alzheimer
Utilisation de 6RF pour la diagnostic de M. Alzheimer

(30) Priority: 26.01.1988 US 148667; 13.06.1988 US 206100
(43) Date of publication of application: 02.08.1989
(73) Proprietor: APPLIED RESEARCH SYSTEMS ARS HOLDING N.V., Curacao (AN)
(72) Inventor: Cacabelos, Ramon Avenida de la Coruna, La Coruna (ES); Vila, Francisco Rubia, Madrid (ES); Alonso, Thomas Ortiz, E-28015 Madrid (ES)
(74) Representative: Jones, Helen Marjorie Meredith

(56) References cited:
- EP-A- 0 215 171
- DE-A- 3 424 279
- Abstract (from the Abstract book) of the lecture of Cacabelos at the Madrid Somatostatin Symposium (7-8 0ctober 1986) of the E.N.E.A.
- THE NEW ENGLAND JOURNAL OF MEDICINE vol. 312, no. 11, 1985, page 725; N.R. CUTLER et al.
- NEUROBIOLOGY OF AGING, vol. 3, 1982 pages 61-68, ANKH0 Int. Inc. US; R.T: BARTUS et al.
- HORMONE RES., vol.29, February/March 1988, Symposium 1986, pages 129-132, Karger AG. Inc., DE; R. CACABELOS et al.
- ACTA ENDOCRINOLOGICA, vol. 117, March 1988, pages 295-301; R. CACABELOS et al.
- FED. PROC., vol. 44, 1985, abstract no. 6677; L. RUMENIK et al.
- PHARMACOLOGY BIOCHEMISTRY & BEHAVIOR, vol. 21, 1984, pages 833-837, Ankho Int. Inc. US; L. VECSEI et al.
- PEPTIDES, vol. 4, 1983, pages 293-295, Ankho Int. Inc., US; L. VECSEI et al.
- JPN. J. PSYCHOPHARMACOL., vol. 8, 1988, pages 235-236; R. CACABELOS et al.

## Description

The somatotropinergic system (STS) is the only neuroendocrine axis in which specific stimulatory and inhibitory neuropeptidergic regulators have been demonstrated thus far. In normal conditions, growth hormone releasing factor (GRF) and somatostatin (SS) are the hypothalamic hypophysiotropic hormones responsible for the regulation of growth hormone (GH) secretion.

At the same time, GRF and SS are influenced by central monoaminergic and peptidergic neuromodulators to optimize the function of the STS. See Cacabelos R., Niigawa H. and Hariguchi S., "Hypothalamohypophyseal System and Brain Function", J. Clin. Sci. 22:1108-1120 (1986).

Recent investigations suggest that the functional structure of the STS characteristically represented at the peripheral level might exist in the central nervous system (CNS). Furthermore, SS levels are reduced in specific areas of the CNS in patients with senile dementia and are elevated in the neostriatum of patients with Huntington's chorea. See Cacabelos R., Niigawa H. and Ikemura Y., "Neuroendocrine Correlates in Senile Dementia of the Alzheimer Type", Progr. Clin. Neurosci., 2:231-247 (1986); Beal M.F., Uhl G., Mazurek M.F., Kowall N. and Martin J.B. "Somatostatin: Alterations in the Central Nervous System in Neurological Diseases", in Martin J.B. and Barchas J.D. (Ed.), Neuropeptides in Neurologic and Psychiatric Disease, pp. 215-257 (Raven Press, New York, 1986); Epelbaum J. "Somatostatin in the Central Nervous System: Physiology and Pathological Modifications", Progr. Neurobiol., 27:63-100 (1986). In addition, an abnormal rise of GH in response to GnRH or TRH has been described in several neuropsychiatric disorders. See Brown G.M., Koslow S.M., and Reichlin S., Ed., Neuroendocrinology and Psychiatric Disorder (Raven Press, New York, 1984). It has been speculated that the central regulators of the STS (e.g., GRF, SS) influence higher activities of the CNS. Such speculation led to search for potential therapeutic uses of these neuropeptides in those neurological diseases in which the normal functioning of the STS is impaired.

The neurochemical characteristics of early and late onset senile dementia of the Alzheimer type (SDAT) can be clearly demonstrated in postmortem studies in which it is possible to observe that cholinergic and somatostatinergic deficits are more pronounced in patients with an early onset of the disease. See Rossor M.N., Iversen L.L., Reynolds G.P., Mountjoy C.Q. and Roth M. "Neurochemical Characteristics of Early and Late Onset Types of Alzheimer's Disease", Br. Med. J., 288:961-964 (1984). In the advanced stages, clinical assessments show that the course of the disease is more rapid and prominent in younger patients.

Since dementia may be attributed to various etiologies of which SDAT accounts for at least 50% of the cases, the search for antemortem markers capable of establishing an early differential diagnosis for identifying potentially reversible or treatable causes of dementia is becoming an extremely important matter.

SDAT is believed to be a multisystem disorder. See Price D.L., Struble R.G., Whitehouse P.J., Kitt C.A., Cork L.C., Walker L.C. and Casanova M.F. "Alzheimer's Disease: A Multisystem Disorder" in: Martin J.B. and Barchas J.D. (Ed), Neuropeptides in Neurologic and Psychiatric Disease, pp. 209-214 (Raven Press, New York, 1986). The most relevant peptidergic abnormalities in SDAT involve a clear deficit in cortical somatostatin and corticotropin-releasing factor (CRF). Other peptidergic and monoaminergic systems are also affected.

It has been discovered that the hypothalamic hypophysiotropic hormones, that is growth hormone releasing factor and somatostatin, affect mental function. Specifically, either or both hormones exert significant affects on locomotor activity and improve learning abilities. When the individual is suffering from a neuropsychiatric disorder, the hormones can induce at least partial return toward normalcy. When the individual is not suffering from a neuropsychiatric disorder, e.g. in healthy individuals, the hormones can effect improvement of the mental function. For instance, significant changes have been observed in such psychometric tests as short memory and attention tests, as well as in EEG (electroencephalogram mapping). Also because the hormones effect an improvement in individuals suffering from senile dementia of the Alzheimer's type, they can be used to diagnose this deficiency, particularly un the early stages. In the pre-conference abstract of a lecture to be given by Cacabelos et al at the conference of the European Neuroendocrine Association in Madrid on 7-8 October 1986 it was disclosed that growth hormone releasing hormone had an effect on learning in rats with lesiens in the nucleus basilis of Meynert (NBM) and a suggestion was made that that agent may have clinical implications for SDAT patients. There is no further explanation of such implications in the abstract however.

The inventors have now found that the GRF induced GH response can be taken as a useful marker for early stages of SDAT.

In the present invention there is provided the new use of growth hormone releasing factor or a physiologically active fragment thereof in the manufacture of a composition for the diagnosis of senile dementia of the Alzheimer's type.

Various commercially available forms of GRF can be employed in the invention. Biologically active fragments can be substituted for all or part of the GRF used.

Growth hormone releasing factor is usually described as the stimulatory growth-hormone releasing factor of the hypothalamus that assists in the neuro-regulation of growth hormone secretion. It is compound NO. 4416 in Merck Index, 10th Ed. (1983). Preferred for use herein are the segments or fragments designated as 1-44 and 1-29. Various commercial products containing useful forms may be used herein. Mixtures are operable.

Pharmaceutical preparations used in accordance with the invention will contain one or more of the hormones together with the conventional pharmaceutically acceptable vehicles. Peptide levels of approximately 1 to 10 »g/kg body weight i.v. and of about 5 to approximately 50 »g/kg body weight s.c. are operable.

Figure 1 shows GRF-induced GH response in elderly subjects (●) and patients with early (■) and late onset senile dementia of the Alzheimer type (▲).
* p<0.005 vs. basal level (0) (means ± SD)
** p<0.005 vs. control ( )
Figure 2 illustrates the correlation between the GRF-induced GH response 60 minutes after injection and the mental performance of patients with early onset senile dementia of the Alsheimer type 24 hours prior to testing.

### Example

The subjects were divided into three groups:
(a) controls (N = 9): 5 females and 4 males (age = 70.10 ± 2.76 years; range = 66-75);
(b) inpatients with early onset senile dementia of the Alzheimer type (EOSDAT) (N = 10): 5 females and 5 males (age 65.00 ± 3.31 years; range = 57-69); and
(c) inpatients with late onset senile dementia of the Alzheimer type (LOSDAT) (N = 10): 5 females and 5 males (age = 75.70 ± 3.77 years; range = 70-82).

All the SDAT patients met the DSM-III criteria for primary degenerative dementia and rigorous diagnostic criteria for SDAT. As EOSDAT patients were considered those whose insidious memory loss or deterioration of cognitive functions began before the age of 60 and had a progressive course of more than 2 years. In LOSDAT, the onset of the disease occurred after the age of 60. The degree of severity of the dementia was assessed with Hasegawa's Dementia Rating Scale (DRS) (Hasegawa K. and Inoue K., "An Investigation of Dementia Rating Scale for the Elderly", Seishinigaku 16:965-969, 1974), Folstein's Mini-Mental State (MMS) Test (Folstein M.F., Folstein S. and McHugh P., "Mini-Mental State: A Practical Method for Grading the Cognitive State of Patients for the Clinician., J. Psychiat. Res. 12:189-198, 1975) and a modified model of the Brief Cognitive Rating Scale (BCRS) and Functional Assessment Stages (FAST) of Reisberg (Reisberg B., Ferris S.H. and De Leon M.J. "Senile Dementia of the Alzheimer Type: Diagnosis and Differential Diagnostic Features with Special Reference to Functional Assessment Staging" in Traber J. and Gispen W.H. (Ed), Senile Dementia of the Alzheimer Type, pp. 18-37, Springer-Verlag_{,} Berlin, 1985). Concomitant pathology other than SDAT was discarded under a careful screening of the subjects with CT Scan, EEG, ECG and complementary laboratory data. Current diagnosis or history of SDM-III major affective disorder or schizophrenia as well as history of endocrine disease were criteria for exclusion. All of the control subjects were free of psychiatric and endocrine illness. All subjects included had been drug-free for a minimum of 7 days before testing.

A 21-gauge indwelling venous needle with an attached three-way stopcook was inserted into the antecubital vein. Baseline blood samples for GH determination were collected immediately before testing. GRF(1-44)NH₂ was then injected as an intravenous bolus (100 »g), and blood samples were obtained at 15, 30, 45, 60, 90 and 120 minutes after injection for determination of GH concentration in plasma. The plasma GH levels were measured by radioimmunoassay using a commercial GH-RIA-Kit (Dainabot Co. Ltd. Tokyo). The intra- and inter-assay coefficients of variation were 6.9% and 10.6%, respectively.

Simultaneously to the performance of the GRF test, electroencephalographic and cardiovascular monitorization of the subjects were carried out. Twenty-four hours prior to the GRF test, and 3, 12, 24 and 48 hours after testing, the DRS, MMS, BCRS, and FAST tests were performed to evaluate mental performance and behavior. Additional monitoring of GRF effects was done by global physician assessment and evaluation of the Mihara Nursing Scale (MNS). This test was developed to be used as a non-cognitive parametric indicator involving several items, including quantification of food intake, locomotion, social interaction, and complementary laboratory data.

The results were analyzed statistically by the Student's test, analysis of variance (ANOVA) and the Mann-Whitney test using a NEC PC-9801VM2 computer.

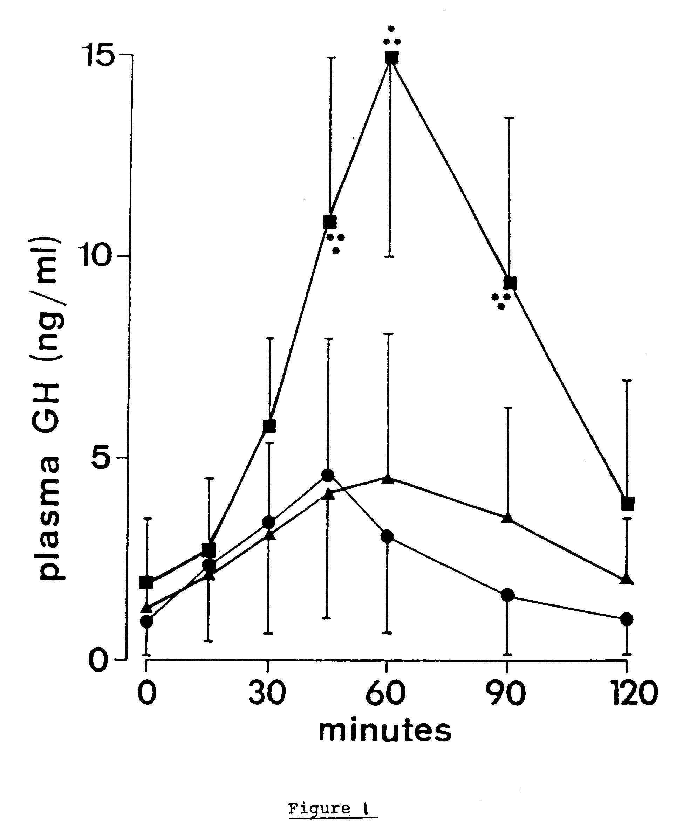

GRF inducted a marked increase in the levels of plasma GH in from 30 to 90 minutes after injection with a maximum peak (15.61 ± 5.71 ng/ml, t = 6.08, p<0.005) at 60 minutes in EOSDAT (Fig. 1). This response was absent in both LOSDAT and control subjects, in whom plasma GH increased slightly but never reached a significant level over basal concentrations (Fig. 1).

In the EOSDAT responders, the rise of plasma GH levels after GRF injection showed a great individual variability (range = 5.5-28.75 ng/ml). This response was higher in females (19.74 ± 4.86 ng/ml) than in males (11.52 ± 2.82 ng/ml, p<0.005) (see the Table). No sexual differences were evident in the other two groups. Significant differences in the basal plasma GH concentrations among the three groups were not detected.

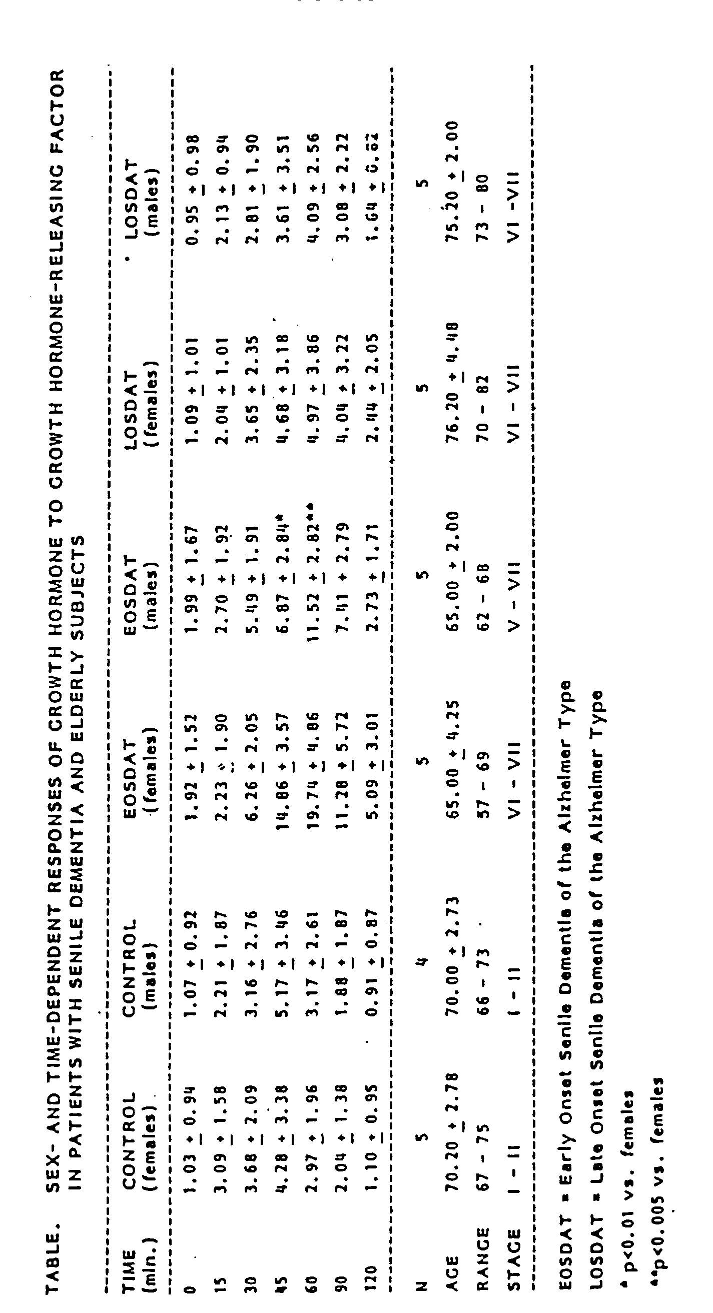

EEG was abnormal in 95% if SDAT patients due to the advanced stage of the disease. Basic electro-encephalographic patterns for controls (N = 6) and SDAT subjects (N = 7) prior to the GRF test were 9.27 ± 0.11 Hz/47.36 ± 3.36 uV and 8.08 ± 0.67 Hz/40.80 ± 6.06 uV, respectively. Global EEG evaluation after testing yielded a basic rhythm of 9.05 ± 0.12 Hz (p<0.025)/49.67 ± 5.75 uV for controls and 7.46 ± 0.53 Hz/43.69 ± 5.12 uV for SDAT. Sequential EEG analysis revealed a fall in frequency (8.75 ± 0.25 Hz, p<0.005) and an increase in amplitude (55.60 ± 6.40 uV, p<0.02) in control subjects and in SDAT patients (7.05 ± 0.90 Hz, p<0.05/52.5 ± 9.89 uV, p<0.005) in from 15 to 45 minutes after injection, preceding the maximum plasma GH peak.

There was a good correlation of scores between the DRS and MMS tests. Basal scores for DRS/MMS prior to the GRF test were 3.5 ± 1.75/3.5 ± 2.65. After testing, scores increased to 5.5 ± 2.10/5.15 ± 3.75 (3 hr), 6.75 ± 3.25/5.5 ± 3.65 (12 hr), 4.35 ± 1.95/4.5 ± 2.75 (24 hr), and 3.5 ± 1.55/4.35 ± 2.75 (48 hr).

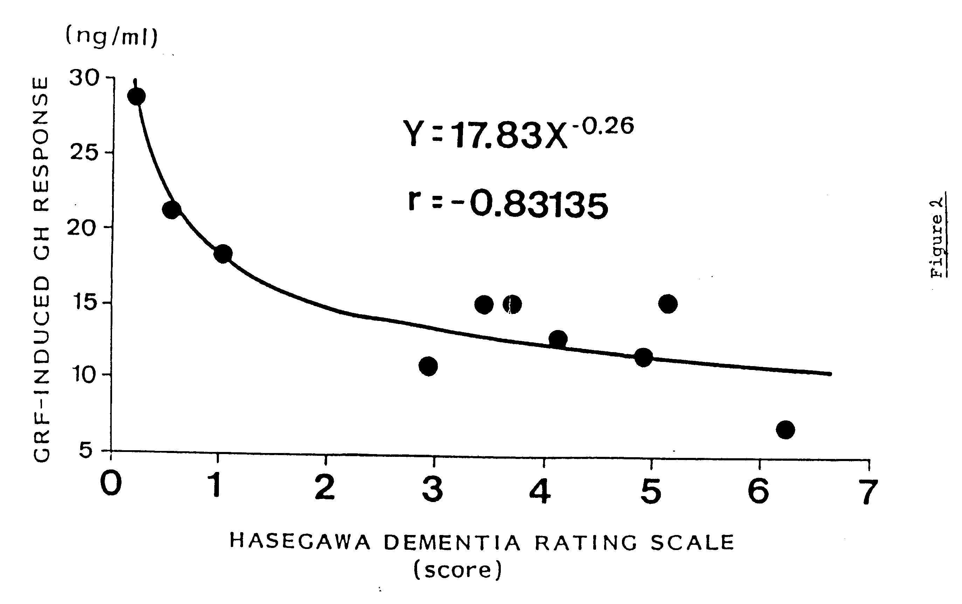

Although these differences were not statistically significant, they were clinically relevant. An inverse curvilinear relationship (Fig. 2) between the variables mental performance (x) and maximum GH response to GRF at 60 minutes (y) was observed (r = - 0.83). A careful evaluation of the BCRS showed that the axes more significantly affected by GRF were Axis 4 (orientation, 30%), Axis 7 (psychomotor, 50%), and Axis 8 (mood and behavior, 40%). According to the MNS, appetite increased in 45% of the patients and in 30% of the controls (2 patients developed a bulimic syndrome), and social interaction improved in 40% of the patients. All these behaviors were transient.

These results demonstrate that GRF induces a significant increase of the plasma GH levels 60 minutes after injection in EOSDAT. This GH response is about 15 minutes delayed with respect to the small peak observed in control subjects.

Furthermore, the GRF-induced GH response slightly correlates with the severity of the disease and is accompanied by EEG and behavioral changes mainly circumscribed to psychomotor functions.

## Claims

1. Use of growth hormone releasing factor or a physiologically active fragment thereof in the manufacture of a composition for the diagnosis of senile dementia of the Alzheimer's type.

2. The use according to claim 1, which comprises administering to an individual an amount of the growth hormone releasing factor effective to enhance the mental function of an healthy individual and monitoring the neuropsychiatry and growth hormone response.

3. The use of any preceding claim wherein the composition is suitable for an administration of growth hormone releasing factor in an amount from about 1 to 50 »g/kg.

4. The use of any preceding claim wherein the composition is suitable for intraveneous administration of growth hormone releasing factor in an amount from 1 to 10 »g/kg.

5. The use of any of claims 1 to 3 wherein the composition is suitable for subcutaneous administration of growth hormone releasing factor in an amount of 5 to 50 »g/kg.

## Patentansprüche

1. Verwendung von Wachstumshormon-freisetzendem Faktor oder einem physiologisch aktiven Fragment davon bei der Herstellung einer Zusammensetzung für die Diagnose von Alters-Demenz vom Alzheimer-Typ.

2. Verwendung nach Anspruch 1, welche umfaßt die Verabreichung einer zur Steigerung der mentalen Funktion einer gesunden Person effektiven Menge des Wachstumshormonfreisetzenden Faktors an eine Person und die Überwachung der Neuropsychiatrie und des Wachstumshormon-Ansprechens.

3. Verwendung nach irgendeinem vorangehenden Anspruch, in welcher die Zusammensetzung für eine Verabreichung von Wachstumshormon-freisetzendem Faktor in einer Menge von etwa 1 bis 50 »g/kg geeignet ist.

4. Verwendung nach irgendeinem vorangehenden Anspruch, in welcher die Zusammensetzung für die intravenöse Verabreichung von Wachstumshormon-freisetzendem Faktor in einer Menge von 1 bis 10 »g/kg geeignet ist.

5. Verwendung nach irgendeinem der Ansprüche 1 bis 3, in welcher die Zusammensetzung für die subkutane Verabreichung von Wachstumshormon-freisetzendem Faktor in einer Menge von 5 bis 50 »g/kg geeignet ist.

## Revendications

1. Utilisation du facteur de libération de l'hormone de croissance ou d'un de ses fragments physiologiquement actifs dans la fabrication d'une composition pour le diagnostic de la démense sénile du type Alzheimer.

2. Utilisation selon la revendication 1, qui comprend l'administration à un individu d'une quantité du facteur de libération de l'hormone de croissance efficace pour augmenter la fonction mentale d'un individu sain et l'enregistrement de la réponse de la GH et des réponses neuropsychiatriques.

3. Utilisation selon une quelconque des revendications qui précèdent, dans laquelle la composition est appropriée pour l'administration du facteur de libération de l'hormone de croissance dans une quantité allant d'environ 1 à 50 »g/kg.

4. Utilisation selon une quelconque des revendications qui précèdent, dans laquelle la composition est appropriée pour l'administration intraveineuse du facteur de libération de l'hormone de croissance dans une quantité allant de 1 à 10 »g/kg.

5. Utilisation selon une quelconque des revendications 1 à 3, dans laquelle la composition est appropriée pour l'administration du facteur de libération de l'hormone de croissance par voie sous-cutanée dans une quantité de 5 à 50 »g/kg.
